# EUROPÄISCHE PATENTANMELDUNG

(11) **EP 0 741 994 A1**
(43) Veröffentlichungstag der Anmeldung: **13.11.1996**
(21) Anmeldenummer: 95890092.0
(22) Anmeldetag: 11.05.1995
(51) Int. Cl.: A61B 5/06, A61B 6/14

(54) **Verfahren zur Darstellung des Kiefers**

(71) Anmelder: TRUPPE, Michael, Dr., 1110 Wien (AT)
(72) Erfinder: TRUPPE, Michael, Dr., 1110 Wien (AT)
(74) Vertreter: Babeluk, Michael, Dipl.-Ing. Mag., Patentanwälte Babeluk - Krause

(57) **Zusammenfassung**

Die Erfindung betrifft ein Verfahren zur Darstellung des Kiefers (1, 2) einer Person und eines Modells davon. Das Verfahren besteht aus folgenden vorbereitenden Schritten:
- Einführen einer Vorrichtung (3) zur Lagebestimmung, die Markierungspunkte (4) aufweist, in die Mundhöhle der Person;
- Erstellen mindestens einer Aufnahme des Kiefers (1, 2) der Person mit einem bildgebenden Verfahren, wie etwa Röntgen, Tomographie, NMR oder dgl., wobei die Markierungen mit aufgenommen werden, und Speicherung der Aufnahme als Datensatz;
- Identifikation der Markierungspunkte (4);
   Herstellen eines Abdruckes des Kiefers (1, 2) und Erzeugen eines Modells des Kiefers (1, 2).
Zur Darstellung werden folgende Schritte durchgeführt:
- Anbringen eines weiteren 3D-Sensors auf der Außenseite des betreffenden Kiefers (1, 2);
- Neuerliches Einführen der Vorrichtung (3) zur Lagebestimmung, falls diese inzwischen entnommen worden ist, in die Mundhöhle in gleicher Lage wie bei der Erstellung der Aufnahme, wobei die Vorrichtung (3) mit einem 3D-Sensor ausgestattet ist;
- Bestimmen der lagemäßigen Beziehung zwischen dem 3D-Sensor der Vorrichtung (3) und dem 3D-Sensor auf der Außenseite des Kiefers (1, 2);
- Entfernen der Vorrichtung (3) zur Lagebestimmung;
- Anbringen eines weiteren 3D-Sensors auf der Außenseite des Modells des Kiefers (1, 2);
- Einführen der Vorrichtung (3) zur Lagebestimmung in das Modell des Kiefers (1, 2) in gleicher Lage wie bei der Erstellung der Aufnahme, wobei die Vorrichtung (3) mit einem 3D-Sensor ausgestattet ist;
- Bestimmen der lagemäßigen Beziehung zwischen dem 3D-Sensor der Vorrichtung (3) und dem 3D-Sensor auf der Außenseite des Modells des Kiefers (1, 2);
- Entfernen der Vorrichtung (3) zur Lagebestimmung;
- Erzeugen einer Überlagerung eines optischen Bildes des Kiefers (1, 2) mit dem Datensatz in lagerichtiger Zuordnung oder alternativ dazu Erzeugen einer Überlagerung eines optischen Bildes des Modells des Kiefers (1, 2) mit dem Datensatz in lagerichtiger Zuordnung. Weiters betrifft die Erfindung eine Vorrichtung (3) zur Lagebestimmung.

## Beschreibung

Die Erfindung betrifft ein Verfahren zur Darstellung des Kiefers einer Person und/oder eines Modells davon.

Bei der Herstellung von zahnärztlichen Implantaten ist eine genaue Planung des operativen Eingriffes erforderlich. Dazu stehen verschiedene Maßnahmen zur Verfügung. In der Regel wird ein Abdruck des betreffenden Kiefers des Patienten hergestellt, woraus wiederum ein Modell des Kiefers gewonnen werden kann. Andererseits sind verschiedene bildgebende Verfahren dazu geeignet, dem behandelnden Arzt Aufschluß über die jeweilige Situation zu verschaffen. Es kann sich dabei um gewöhnliche Röntgenbilder handeln, um Tomographien oder um NMR's (Kernspintomographie). Unbefriedigend dabei bleibt jedoch, daß das Modell einerseits und die Darstellung aus dem bildgebenden Verfahren andererseits erst durch den Betrachter in Übereinstimmung gebracht werden müssen. Noch schwieriger ist die Aufgabe, die reale Situation bei der Operation selbst mit dem Modell bzw. mit der zuvor erstellten Darstellung in Übereinstimmung bringen zu müssen.

Aus der EPA 488 987 des Anmelders ist ein Verfahren zur Darstellung beweglicher Körper bekannt, bei dem eine optische Darstellung des Körpers und ein dem Körper zugeordnetes Datenfeld gleichzeitig oder alternativ aus derselben Perspektive und im selben Maßstab in Echtzeit dargestellt werden. Ein solches Verfahren ist grundsätzlich dazu geeignet, die oben beschriebene Problematik zu lösen. Es wird etwa auf einem Monitor ein optisches Bild des betreffenden Kiefers angezeigt und gleichzeitig wird der zugehörige Datensatz, das ist das Ergebnis des bildgebenden Verfahrens, angezeigt. An den betreffenden Körperteilen ist dabei ein Lagesensor anzubringen, der gewährleistet, daß sich der Datensatz entsprechend einer allfälligen Bewegung des Körperteils, z.B. des Unterkiefers im vorliegenden Fall, verschiebt. Voraussetzung für dieses Verfahren ist jedoch eine anfängliche Einstellung des Systems, bei der charakteristische Punkte des Datensatzes identifiziert werden und mit den entsprechenden charakteristischen Punkten in der optischen Darstellung in Übereinstimmung gebracht werden. Gerade bei der Anwendung eines solchen Verfahrens auf das menschliche Kiefer stellen sich hierbei jedoch große Probleme, da die erforderlichen charakteristischen Punkte nicht in ausreichender Zahl vorhanden sind. Dies trifft inbesonders dann zu, wenn es sich um einen zahnlosen Kiefer handelt. Außerdem macht die benötigte Genauigkeit im Bereich des Kiefers eine solche Einstellung auch bei Vorliegen von Zähnen problematisch.

Es sind weiters sogenannte Artikulatoren zur Bestimmung der Lage des Unterkiefers bekannt. Damit kann beispielsweise die Lage der virtuellen Längsachse des Unterkiefers bestimmt werden. Solche Artikulatoren sind jedoch nicht dazu geeignet, das oben beschriebene Problem zu lösen, da die Mundhöhle beim Eingriff selbst frei zugänglich sein muß.

Eine weiterhin mögliche Anbringung von Markierungspunkten auf der Haut an der Außenseite des Kiefers ist nicht durchführbar, da die Patienten während des Eingriffs normalerweise narkotisiert werden. Dazu werden sie durch die Nase intubiert, wodurch sich eine völlige Verspannung der Gesichtsmuskulatur ergibt, was zu einer Verschiebung der Markierungspunkte führt. Dies macht ein Heranziehen dieser Punkte zur Lagebestimmung unmöglich. Ein Befestigung eines Markierungskörpers durch die Haut hindurch bereits bei der Aufnahme vorzunehmen, ist wegen der damit verbundenen Belastung für den Patienten im allgemeinen nicht möglich.

Eine Aufgabe der Erfindung ist es, diese Nachteile zu vermeiden und ein Verfahren zu schaffen, das eine sorgfältige Planung eines operativen Eingriffes im Bereich der Kiefer anhand eines Modells ermöglicht, wobei die Darstellungen aus bildgebenden Verfahren in anschaulicher Weise einbezogen werden können Ein weitere Aufgabe der Erfindung ist es, während eines operativen Eingriffes selbst eine Kontrolle des jeweils erreichten Zustandes zu ermöglichen, indem eine optische Darstellung des Operationsbereiches zur Verfügung gestellt wird, die lagerichtig mit dem Ergebnis des bildgebenden Verfahrens überlagert ist.

Erfindungsgemäß wird dies in einem ersten Aspekt der Erfindung durch die Ausführung folgender Schritte erreicht:
1. vorbereitende Schritte: diese können zu einem beliebigen Zeitpunkt vor der eigentlichen Darstellung durchgeführt werden, wobei lediglich gefordert werden muß, daß sich der Kiefer zwischen der Durchführung dieser Schritte und der Darstellung nicht verändert, etwa durch einen zahnärztlichen Eingriff.
   - Einführen einer Vorrichtung zur Lagebestimmung, die Markierungspunkte aufweist, in die Mundhöhle der Person; wesentlich an der Vorrichtung ist, daß sie eine eindeutig und genau reproduzierbare Stellung in der Mundhöhle einnimmt. Die Markierungspunkte sollen in dem jeweils verwendeten bildgebenden Verfahren einen guten Kontrast ergeben und somit leicht identifizierbar sein. Es kann sich im Fall von Röntgenaufnahmen etwa um Bleikügelchen handeln, die fest in Kunststoffmaterial eingegossen sind. Als Markierungspunkte können auch die Ecken einer in der Vorrichtung eingebauten Drahtstruktur o. dgl. dienen. Der in der Folge beschriebene Lagesensor selbst ist zu diesem Zeitpunkt nicht notwendigerweise in der Vorrichtung enthalten oder an ihr angebracht. Wesentlich ist jedoch, daß dieser Lagesensor in der Folge lagemäßig eindeutig mit der Vorrichtung verbindbar ist.
   - Erstellen mindestens einer Aufnahme des Kiefers der Person mit einem bildgebenden Verfahren, wie etwa Röntgen, Tomographie, NMR oder dgl., wobei die Markierungen mit aufgenommen werden, und Speicherung der Aufnahme als Datensatz; diese sogenannte Digitalisierung ist eine Voraussetzung für die spätere Bearbeitung der Aufnahme.
   - Identifikation der Markierungspunkte; diese kann von Hand durchgeführt werden, etwa indem ein Markierungspunkt nach dem anderen auf einem Bildschirm mit dem Cursor aufgesucht wird und dadurch identifiziert wird. Dies bedeutet, daß die Koordinaten der Markierungspunkte im Koordinatensystem der Aufnahme festgestellt werden. Es ist jedoch auch durchaus möglich, ein Mustererkennungsverfahren zur Identifikation zu verwenden.
2. Schritte zur Durchführung der eigentlichen Darstellung: diese Schritte werden anläßlich einer Untersuchung der Person oder während eines Eingriffs durchgeführt.
   - Anbringen eines 3D-Sensors auf der Außenseite des betreffenden Kiefers; dies erfolgt beispielsweise dadurch, daß die Halterung für den 3D-Sensor durch die Haut im Bereich des Kinns der Person eingeschraubt wird, wenn die Darstellung des Unterkiefers erfolgen soll. Es ist selbstverständlich auch möglich, sowohl Unterkiefer als auch Oberkiefer für die Darstellung vorzubereiten, indem zwei Sensoren verwendet werden.
   - Neuerliches Einführen der Vorrichtung zur Lagebestimmung, falls diese inzwischen entnommen worden ist, in die Mundhöhle in gleicher Lage wie bei der Erstellung der Aufnahme, wobei die Vorrichtung mit einem 3D-Sensor ausgestattet ist; Es ist im Prinzip möglich, daß die Aufnahme unmittelbar vor der Darstellung hergestellt wird. In diesem Fall kann sich die Vorrichtung zur Lagebestimmung noch in der Mundhöhle befinden. Im allgemeinen wird jedoch in diesem Schritt die Vorrichtung neu eingesetzt, wobei darauf zu achten ist, daß exakt die gleiche Lage in Bezug auf das Kiefer wie bei der Aufnahme erreicht wird. Nunmehr muß der 3D-Sensor an der Vorrichtung angebracht sein oder in ihr eingebaut sein. Wesentlich ist, daß die Lage dieses Sensors in Bezug auf die Markierungen eindeutig ist und genau bekannt ist.
   - Bestimmen der lagemäßigen Beziehung zwischen dem 3D-Sensor der Vorrichtung und dem 3D-Sensor auf der Außenseite des Kiefers; dies erfolgt dadurch, daß die räumliche Lage der beiden Sensoren gleichzeitig oder nahezu gleichzeitig bestimmt wird.
   - Entfernen der Vorrichtung zur Lagebestimmung; dadurch wird der freie Zugang zur Mundhöhle hergestellt.
   - Erzeugen einer Überlagerung eines optischen Bildes des Kiefers mit dem Datensatz in lagerichtiger Zuordnung. Durch den Sensor auf der Außenseite des Kiefers ist die aktuelle Lage des Kiefers stets bekannt. Durch die Durchführung einer Reihe von Koordinatentransformationen gelingt es, den Datensatz so zu positionieren, daß die Strukturen des Datensatzes mit den entsprechenden Strukturen des optischen Bildes auch bei einer räumlichen Bewegung des Kiefers stets in Übereinstimmung bleiben. Wenn beide Kiefer mir Sensoren versehen sind, kann zwischen der lagerichtigen Darstellung des Unterkiefers und des Oberkiefers umgeschaltet werden. Es ist jedoch auch möglich, den Datensatz in einen das Unterkiefer betreffenden Teil und einen das Oberkiefer betreffenden Teil zu unterteilen, wobei es dann möglich ist, sowohl Unterkiefer als auch Oberkiefer lagerichtig mit dem entsprechenden Datensatz überlagert darzustellen.

Wesentlich an der vorliegenden Erfindung ist, daß der Schritt der anfänglichen Einstellung der Zuordnung zwischen dem Datensatz und der optischen Darstellung wesentlich vereinfacht wird. Wenn diese Zuordnung einmal hergestellt ist, kann das Verfahren so ablaufen, wie dies in der EPA 488 987 beschrieben ist. Da die Lage der Markierungspunkte in der Vorrichtung zur Lagebestimmung bekannt ist, ist es nicht notwendig, diese Punkte mit einem 3D-Stylus abzutasten, oder sonstige Maßnahmen zu treffen, um das System einzustellen.

In einem zweiten Aspekt der vorliegenden Erfindung wird die Darstellung nicht mit dem betreffenden Kiefer selbst durchgeführt, sondern es wird ein Modell der Kiefer in einer bekannten Art hergestellt. Die Vorrichtung zur Lagebestimmung paßt dann auch in dieses Modell in einer eindeutig bestimmten Lage. Auf diese Weise kann das Modell mit lagerichtig überlagertem Datensatz dargestellt werden. Damit ist es in einer sehr komfortablen Art möglich, eine Operationsplanung durchzuführen, oder verschiedene Varianten eines Eingriffs zu simulieren.

Diese beiden ersten Aspekte können miteinander kombiniert werden, wodurch der Bezug des realen Kiefers zu dem Modell in einer sehr anschaulichen Art dargestellt werden kann. Weiters kann die optische Darstellung auch festgehalten werden, indem Photographien oder Videoaufnahmen angefertigt werden.

Die Darstellung selbst kann in der Weise erfolgen, die in der EP-A 488 987 beschrieben ist. Dabei wird eine Videokamera, die mit einem Lagesensor versehen ist, dazu verwendet, eine optische Darstellung des Objektes, also des Kiefers oder des Modells zu erzeugen, die auf einem entsprechenden Monitor angezeigt wird. Da sowohl die räumliche Lage der Kamera, als auch die räumliche Lage des Objektes bekannt ist, kann eine Darstellung des Datensatzes berechnet werden, die dem gleichen Blickwinkel und dem gleichen Maßstab entspricht. Somit fallen die entsprechenden Strukturen der optischen Darstellung und aus der Aufnahme des bildgebenden Verfahrens zusammen und können lagerichtig gleichzeitig oder alternativ dargestellt werden.

Es ist jedoch in einer vorteilhaften Ausführungsvariante der Erfindung möglich, daß das Erzeugen einer Überlagerung des optischen Bildes mit dem Datensatz dadurch erfolgt, daß eine Anzeige des Datensatzes zwischen dem Auge des Betrachters und dem darzustellenden Kiefer bzw. dem Modell des Kiefers zwischengeschaltet wird. Eine solche Anzeige kann etwa als transparentes LCD (Flüssigkristall) Display ausgebildet sein. Der Anwender beobachtet dabei die reale Situation durch das Display hindurch, auf dem gleichzeitig der Datensatz dargestellt wird. Im Auge des Anwenders kommt es dadurch zur Überlagerung. Im allgemeinen Fall ist sowohl der Anwender als auch das Display frei beweglich, wobei jedoch die Randbedingung erfüllt sein muß, daß der Anwender das Objekt durch das Display hindurch beobachten kann. In diesem Fall trägt sowohl der Anwender als auch das Display einen 3D-Sensor, damit wiederum die jeweilige Perspektive berechnet werden kann. Ein solches Verfahren ist für allgemeine Objekte im AT - Patent 398 163 des Anmelders dargestellt. Diese Variante besitzt den Vorteil, daß der Anwender - abgesehen von dem sehr kleinen und leichten Sensor - keinerlei Vorrichtung auf dem Kopf tragen muß. Weiters ist es auf einfache Weise möglich, auch weiteren Personen den Zugang zu der Darstellung zu verschaffen.

Ferner ist es jedoch im Rahmen der Erfindung in gleicher Weise möglich, das Display als Brille auszubilden oder sonst fest mit dem Kopf des Anwenders zu verbinden. Auch die Verwendung halbdurchlässiger Spiegel oder sonstiger Verfahren zur Einspiegelung in das Auge des Betrachters sind grundsätzlich möglich.

In einer besonders bevorzugten Variante des erfindungsgemäßen Verfahrens ist vorgesehen, daß vom Anwender dreidimensionale Strukturen in den Datensatz der Aufnahme eingefügt werden und daß diese in lagerichtiger Zuordnung zum optischen Bild dargestellt werden. Auf diese Weise ist es nicht nur möglich, gewisse nur undeutliche Strukturen in der Aufnahme hervorzuheben, sondern es kann auch in sehr einfacher Weise eine Simulation durchgeführt werden. Dies ist etwa dann vorteilhaft, wenn im Rahmen eines kieferorthopädischen Eingriffs eine Veränderung gezielt herbeigeführt werden soll. Dabei kann der gewünschte oder geplante Endzustand eingezeichnet werden. Es können auch Situationen dargestellt werden, die sich nach dem Entfernen oder Hinzufügen von Knochenkeilen ergeben. Anhand der Darstellung am Modell kann so über die Durchführbarkeit und Problematik eines solchen Eingriffs sehr genau Aufschluß gewonnen werden.

Weiters kann die optische Darstellung nicht nur "live" erfolgen, sondern auch festgehalten werden, indem Photographien oder Videoaufnahmen angefertigt werden. Wenn dabei die Vorrichtung zur Lagebestimmung auch optisch sichtbare Markierungspunkte aufweist, so kann das erfindungsgemäße Verfahren geringfügig modifiziert werden. Die vorbereitenden Schritte entsprechen denen des oben beschriebenen Verfahrens. In der Folge wird der Einfachheit halber von der Aufnahme von Photos ausgegangen. Für Videoaufnahmen gilt das Gesagte sinngemäß. Je nachdem, ob die Photoaufnahmen vom Kiefer selbst oder von einem Modell angefertigt werden soll, wird ein solches Modell hergestellt oder nicht. Wesentlich ist, daß mindestens eine Aufnahme, besser jedoch zwei oder mehr Aufnahmen jeweils aus der gleichen Perspektive einmal mit eingesetzter Vorrichtung zur Lagebestimmung und einmal ohne diese hergestellt werden. Da die Markierungspunkte der Vorrichtung auch auf den Photos sichtbar sind, können diese auf einem Monitor mit der Röntgen-Aufnahme o. dgl. in Übereinstimmung gebracht werden. Auf diese Weise ist es möglich, die entsprechende Koordinatentransformation zu bestimmen. Da die Photos ohne die Vorrichtung aus der gleichen Perspektive hergestellt worden sind, ist es nunmehr möglich, auch in diese den Datensatz aus dem bildgebenden Verfahren lagerichtig einzublenden. Dies ist wesentlich, da erst auf dieser Aufnahme die interessierenden Strukturen ohne Behinderung durch die Vorrichtung ersichtlich sind. Falls zwischen den Aufnahmen der Photos eine gewisse Bewegung des Kiefers stattgefünden hat, wie dies in manchen Fällen unvermeidlich ist, so wird das Verfahren dadurch nicht beeinträchtigt, da der außen angebrachte Lagesensor diese Bewegung erfaßt und somit eine entsprechende Korrektur möglich ist. Neben der Darstellung der Photos ist natürlich auch bei dieser Variante des Verfahrens die "live" Darstellung, wie sie oben beschrieben ist, zusätzlich möglich. Das Verfahren ist vereinfacht, da ein eigener 3D-Sensor der Vorrichtung zur Lagebestimmung entbehrlich ist.

Weiters betrifft die vorliegende Erfindung eine Vorrichtung zur Lagebestimmung, die für eines der obigen Verfahren verwendbar ist, wobei die Vorrichtung in die Mundhöhle einer Person einführbar ist und so ausgebildet ist, daß sie durch Oberkiefer und Unterkiefer der Person in einer eindeutig reproduzierbaren Stellung gehalten werden kann. Diese Vorrichtung ist dadurch gekennzeichnet, daß sie Markierungspunkte aufweist, die mit einem bildgebenden Verfahren identifizierbar sind und daß sie weiters einen Lagesensor aufweist, der eine genaue Bestimmung der räumlichen Lage der Vorrichtung ermöglicht. Wie dies oben bereits beschrieben worden ist, kann der Lagesensor entfernbar sein, solange gewährleistet ist, daß bei eingebautem Sensor die räumliche Zuordnung zwischen Sensor und den Markierungspunkten eindeutig und reproduzierbar ist.

Vorzugsweise ist vorgesehen, daß die Vorrichtung aus einem festen Grundkörper besteht, der die Markierungspunkte und den Lagesensor aufweist und daß an diesem Grundkörper Haltekörper aus einem flexiblen, erhärtbaren Material gebildet sind, die sich der Form von Zähnen oder dem Kiefer anpassen. Die Vorrichtung wird dabei zunächst in der Art eines Abdruckes an das Kiefer angepaßt und kann nach dem Erhärten reproduzierbar in der gleichen Lage eingesetzt werden.

In einer Variante der Vorrichtung weist diese Markierungspunkte auf, die sowohl mit einem bildgebenden Verfahren identifizierbar sind, als auch optisch sichtbar sind.

In der Folge wird die Erfindung durch die in den Figuren dargestellten Ausführungsbeispiele näher erläutert. Die Figuren zeigen schematisch:

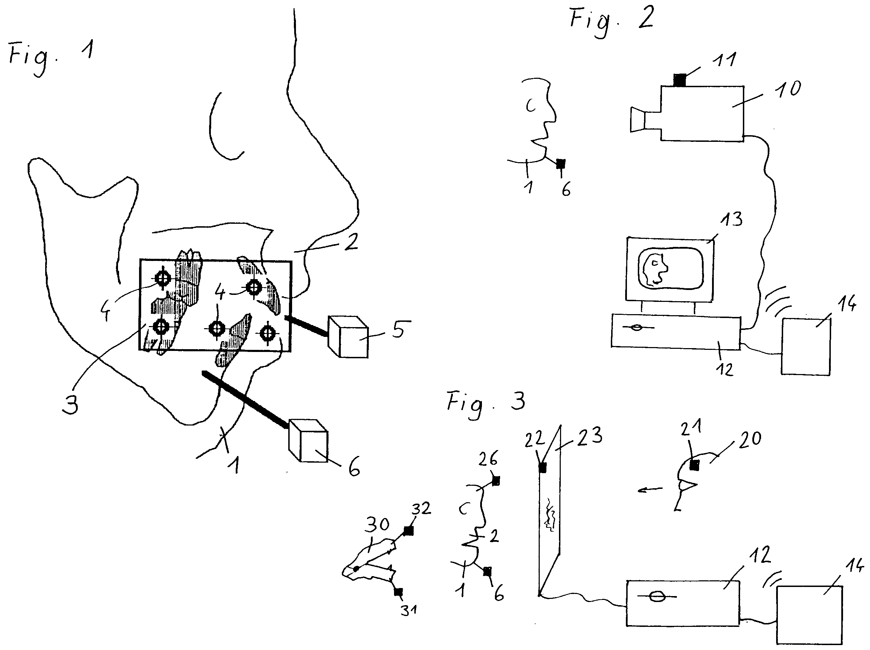

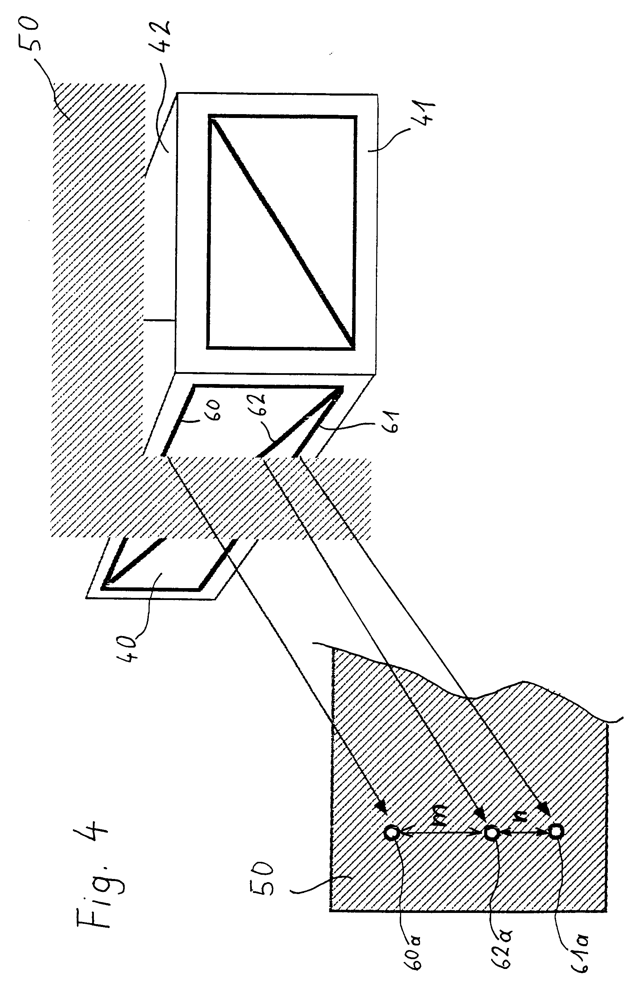

Die Fig. 1 eine Person mit einer in die Mundhöhle eingesetzten Vorrichtung zur Lagebestimmung; die Fig. 2 ein erstes System, mit dem das erfindungsgemäße Verfahren durchgeführt werden kann; die Fig. 3 ein zweites System, mit dem das erfindungsgemäße Verfahren durchgeführt werden kann und die Fig. 4 eine Variante der Ausführung von Markierungspunkten.

In der Fig. 1 ist der Kopf einer Person mit Unterkiefer 1 und Oberkiefer 2 angedeutet. Dazwischen befindet sich eine Vorrichtung 3 zur Lagebestimmung, die als sogenannter Splint ausgebildet ist, der in einer eindeutig reproduzierbaren Stellung eingesetzt werden kann. In dieser Vorrichtung 3 ist eine Anzahl von Markierungspunkten 4 vorgesehen, die z.B. auf einer Röntgendarstellung klar ersichtlich sind. Ein 3D-Sensor 5 ist fest mit der Vorrichtung 3 verbunden. An der Außenseite des Unterkiefers 1 ist ein weiterer 3D-Sensor 6 angeschraubt.

Das System von Fig. 2 besteht aus einer Kamera 10, an der ein 3D-Sensor 11 fest angebracht ist. Die Kamera ist über einen Computer 12 mit einem Monitor 13 verbunden, auf dem der Unterkiefer 1 dargestellt werden kann. In dem Computer 12 ist weiters eine Aufnahme des bildgebenden Verfahrens gespeichert. Weiters ist der Computer mit einem Magnetfeldemitter 14 verbunden, der raumfest angeordnet ist. Dadurch kann die jeweilige räumliche Lage einschließlich der Winkellage der Sensoren 6 und 11 erfaßt werden. Der Computer berechnet in Echtzeit eine Darstellung des Datensatzes aus dem bildgebenden Verfahren und zeigt diese auf dem Bildschirm an.

Bei der Ausführungsvariante von Fig. 3 ist der Computer 12 mit einem durchlässigen LCD-Display 23 verbunden, das einen 3D-Sensor 22 trägt. der Betrachter 20 trägt ebenfalls einen 3D-Sensor 21. Neben dem Sensor 6, der wie bei der vorigen Ausfühzungsvariante mit dem Unterkiefer 1 verbunden ist, ist hier auch ein an der Stirn angebrachter Sensor 26 vorgesehen, der somit mit dem Oberkiefer 2 fest verbunden ist. Der Computer berechnet eine Darstellung des bildgebenden Verfahrens, die der jeweiligen Stellung von Kiefer 1, 2, Display 23 und Betrachter 20 zueinander entspricht. In gleicher Weise kann ein Modell 30 des Kiefers betrachtet werden, das mit Sensoren 31, 32 bestückt ist.

Die Fig. 4 zeigt schematisch eine Alternative zur Ausführung der Markierungspunkte, die in oder an der Vorrichtung zur Lagebestimmung angebracht sind. In drei zueinander rechtwinkeligen Ebenen 40, 41, 42 sind Strukturen angeordnet, die auf der Röntgenaufnahme o. dgl. einen Kontrast ergeben. Zwei dieser Ebenen 40, 42 sind dann, wenn die Vorrichtung 3 in das Kiefer 1, 2 eingesetzt ist, im wesentlichen parallel zur Symmetrieebene des Kopfes. Die Strukturen bestehen jeweils aus einem Rechteck mit einer Diagonale. Mit 50 ist eine gedachte Schichtebene einer CT-Aufnahme bezeichnet, die rechtwinkelig zu den Ebenen 40 und 42 ist. In der entsprechenden Darstellung dieser CT-Schicht 50 erscheint die obere Seite 60 des Rechtecks als Punkt 60a, die untere Seite 61 als Punkt 61a und die Diagonale 62 als zwischen diesen Punkten 60a und 61a liegenden Punkt 62a. Aus dem Verhältnis der Abstände m und n des Punktes 62a zu den Punkten 60a und 61a kann man die genaue Lage der Schicht 50 bestimmen. Auf diese Weise ist es möglich, die Information des CTs über den Tischvorschub zu korrigieren, was etwa dann erforderlich ist, wenn sich der Patient während des Scanvorganges bewegt hat.

Die Ebenen 40, 41, 42 mit den oben beschriebenen Strukturen können sowohl in der Vorrichtung zur Lagebestimmung integriert sein, als auch durch eine starre Verbindung mir ihr so verbunden sein, daß sie außerhalb der Kiefer 1, 2 liegen.

## Patentansprüche

1. Verfahren zur Darstellung des Kiefers einer Person, bestehend aus folgenden vorbereitenden Schritten:
- Einführen einer Vorrichtung (3) zur Lagebestimmung, die Markierungspunkte (4) aufweist, in die Mundhöhle der Person;
- Erstellen mindestens einer Aufnahme des Kiefers (1, 2) der Person mit einem bildgebenden Verfahren, wie etwa Röntgen, Tomographie, NMR oder dgl., wobei die Markierungspunkte (4) mit aufgenommen werden, und Speicherung der Aufnahme als Datensatz;
- Identifikation der Markierungspunkte (4);
wobei zur Darstellung folgende Schritte durchgeführt werden:
- Anbringen eines 3D-Sensors (6, 26) auf der Außenseite des betreffenden Kiefers (1, 2);
- Neuerliches Einführen der Vorrichtung (3) zur Lagebestimmung, falls diese inzwischen entnommen worden ist, in die Mundhöhle in gleicher Lage wie bei der Erstellung der Aufnahme, wobei die Vorrichtung (3) mit einem 3D-Sensor (5) ausgestattet ist;
- Bestimmen der lagemäßigen Beziehung zwischen dem 3D-Sensor (5) der Vorrichtung (3) und dem 3D-Sensor (6, 26) auf der Außenseite des Kiefers (1, 2);
- Entfernen der Vorrichtung (3) zur Lagebestimmung;
- Erzeugen einer Überlagerung eines optischen Bildes des Kiefers (1, 2) mit dem Datensatz in lagerichtiger Zuordnung.

2. Verfahren zur Darstellung eines Modells des Kiefers einer Person, bestehend aus folgenden vorbereitenden Schritten:
- Einführen einer Vorrichtung (3) zur Lagebestimmung, die Markierungspunkte (4) aufweist, in die Mundhöhle der Person;
- Erstellen mindestens einer Aufnahme des Kiefers (1, 2) der Person mit einem bildgebenden Verfahren, wie etwa Röntgen, Tomographie, NMR oder dgl., wobei die Markierungspunkte (4) mit aufgenommen werden, und Speicherung der Aufnahme als Datensatz;
- Identifikation der Markierungspunkte (4);
- Herstellen eines Abdruckes des Kiefers (1, 2) und Erzeugen eines Modells (30) des Kiefers (1, 2);
wobei zur Darstellung folgende Schritte durchgeführt werden:
- Anbringen eines weiteren 3D-Sensors (31, 32) auf der Außenseite des Modells (30) des Kiefers (1, 2);
- Einführen der Vorrichtung (3) zur Lagebestimmung in das Modell (30) in gleicher Lage wie bei der Erstellung der Aufnahme, wobei die Vorrichtung (3) mit einem 3D-Sensor (5) ausgestattet ist;
- Bestimmen der lagemäßigen Beziehung zwischen dem 3D-Sensor (5) der Vorrichtung (3) und dem 3D-Sensor (30, 31) auf der Außenseite des Modells (30) des Kiefers (1, 2);
- Entfernen der Vorrichtung (3) zur Lagebestimmung;
- Erzeugen einer Überlagerung eines optischen Bildes des Modells (30) mit dem Datensatz in lagerichtiger Zuordnung.

3. Verfahren zur Darstellung des Kiefers einer Person und eines Modells davon, bestehend aus folgenden vorbereitenden Schritten:
- Einführen einer Vorrichtung (3) zur Lagebestimmung, die Markierungspunkte (4) aufweist, in die Mundhöhle der Person;
- Erstellen mindestens einer Aufnahme des Kiefers (1, 2) der Person mit einem bildgebenden Verfahren, wie etwa Röntgen, Tomographie, NMR oder dgl., wobei die Markierungspunkte (4) mit aufgenommen werden, und Speicherung der Aufnahme als Datensatz;
- Identifikation der Markierungspunkte (4);
- Herstellen eines Abdruckes des Kiefers (1, 2) und Erzeugen eines Modells (30) des Kiefers (1, 2);
wobei zur Darstellung folgende Schritte durchgeführt werden:
- Anbringen eines weiteren 3D-Sensors (6, 26) auf der Außenseite des betreffenden Kiefers (1, 2);
- Neuerliches Einführen der Vorrichtung (3) zur Lagebestimmung, falls diese inzwischen entnommen worden ist, in die Mundhöhle in gleicher Lage wie bei der Erstellung der Aufnahme, wobei die Vorrichtung (3) mit einem 3D-Sensor (5) ausgestattet ist;
- Bestimmen der lagemäßigen Beziehung zwischen dem 3D-Sensor (5) der Vorrichtung (3) und dem 3D-Sensor (6, 26) auf der Außenseite des Kiefers (1, 2);
- Entfernen der Vorrichtung (3) zur Lagebestimmung;
- Anbringen eines weiteren 3D-Sensors auf der Außenseite des Modells des Kiefers (1, 2);
- Einführen der Vorrichtung (3) zur Lagebestimmung in das Modell (30) des Kiefers (1, 2) in gleicher Lage wie bei der Erstellung der Aufnahme, wobei die Vorrichtung (3) mit einem 3D-Sensor (5) ausgestattet ist;
- Bestimmen der lagemäßigen Beziehung zwischen dem 3D-Sensor (5) der Vorrichtung (3) und dem 3D-Sensor (30, 31) auf der Außenseite des Modells (30) des Kiefers (1, 2);
- Entfernen der Vorrichtung (3) zur Lagebestimmung;
- Erzeugen einer Überlagerung eines optischen Bildes des Kiefers (1, 2) mit dem Datensatz in lagerichtiger Zuordnung oder alternativ dazu Erzeugen einer Überlagerung eines optischen Bildes des Modells (30) des Kiefers (1, 2) mit dem Datensatz in lagerichtiger Zuordnung.

4. Verfahren nach einem der Ansprüche 1 bis 3, dadurch gekennzeichnet, daß das Erzeugen der Überlagerung eines optischen Bildes mit dem Datensatz auf einen Monitor (13) erfolgt, wobei das optische Bild von einer Videokamera (10) aufgenommen wird, die mit einem 3D-Sensor (11) zur Bestimmung der räumlichen Lage fest verbunden ist.

5. Verfahren nach einem der Ansprüche 1 bis 3, dadurch gekennzeichnet, daß das Erzeugen einer Überlagerung des optischen Bildes mit dem Datensatz dadurch erfolgt, daß eine Anzeige des Datensatzes zwischen dem Auge des Betrachters (20) und dem darzustellenden Kiefer (1, 2) bzw. dem Modell (30) des Kiefers (1, 2) zwischengeschaltet wird.

6. Verfahren nach Anspruch 5, dadurch gekennzeichnet, daß die Anzeige als transparentes LCD-Display (23) ausgebildet ist.

7. Verfahren nach Anspruch 5 oder 6, dadurch gekennzeichnet, daß die Anzeige als Brille, als ein Schirm, der zwischen Beobachter und Kiefer (1, 2) bzw. Modell angeordnet ist und der vorzugsweise einen 3D-Sensor trägt, ausgebildet ist, oder daß die Anzeige durch Einspiegelung des Datensatz in das Auge des Beobachters (20) durchgeführt wird.

8. Verfahren nach einem der Ansprüche 1 bis 7, dadurch gekennzeichnet, daß vom Anwender dreidimensionale Strukturen in den Datensatz der Aufnahme eingefügt werden und daß diese in lagerichtiger Zuordnung zum optischen Bild dargestellt werden.

9. Verfahren zur Darstellung des Kiefers einer Person und/oder eines Modells davon, bestehend aus folgenden vorbereitenden Schritten:
- Einführen einer Vorrichtung (3) zur Lagebestimmung, die optisch sichtbare Markierungspunkte (4) aufweist, in die Mundhöhle der Person;
- Erstellen mindestens einer Aufnahme des Kiefers (1, 2) der Person mit einem bildgebenden Verfahren, wie etwa Röntgen, Tomographie, NMR oder dgl., wobei die Markierungspunkte (4) mit aufgenommen werden, und Speicherung der Aufnahme als Datensatz;
- gegebenenfalls Herstellen eines Abdruckes des Kiefers (1, 2) und Erzeugen eines Modells (30) des Kiefers (1, 2);
wobei zur Darstellung folgende Schritte durchgeführt werden:
- Anbringen eines weiteren 3D-Sensors (6, 26) auf der Außenseite des betreffenden Kiefers (1, 2) bzw. des Modells (30) des Kiefers;
- Neuerliches Einführen der Vorrichtung (3) zur Lagebestimmung, falls diese inzwischen entnommen worden ist, in die Mundhöhle bzw. in das Modell (30) in gleicher Lage wie bei der Erstellung der Aufnahme des bildgebenden Verfahrens;
- Herstellung mindestens einer photographischen oder Video-Aufnahme des Kiefers (1, 2) bzw. des Modells (30);
- Entfernen der Vorrichtung (3) zur Lagebestimmung;
- Herstellung mindestens einer weiteren photographischen oder Video-Aufnahme des Kiefers (1, 2) bzw. des Modells (30) aus der gleichen Position;
- Speicherung der photographischen oder Video-Aufnahmen als Datensatz;
- Herstellen der Übereinstimmung der Markierungspunkte auf der Aufnahme des bildgebenden Verfahrens und der optischen Darstellung;
- Erzeugen einer Überlagerung eines optischen Bildes des Kiefers (1, 2) mit dem Datensatz in lagerichtiger Zuordnung oder alternativ dazu Erzeugen einer Überlagerung eines optischen Bildes des Kiefers (1, 2) bzw. des Modells (30) mit dem Datensatz in lagerichtiger Zuordnung.

10. Vorrichtung zur Lagebestimmung, die für ein Verfahren nach einem der Ansprüche 1 bis 8 verwendbar ist, wobei die Vorrichtung (3) in die Mundhöhle einer Person einführbar ist und so ausgebildet ist, daß sie durch Oberkiefer (2) und Unterkiefer (1) der Person in einer eindeutig reproduzierbaren Stellung gehalten werden kann, dadurch gekennzeichnet, daß die Vorrichtung (3) Markierungspunkte (4) aufweist, die mit einem bildgebenden Verfahren identifizierbar sind und daß sie weiters einen Lagesensor (5) aufweist, der eine genaue Bestimmung der räumlichen Lage der Vorrichtung (3) ermöglicht.

11. Vorrichtung nach Anspruch 10, dadurch gekennzeichnet, daß sie aus einem festen Grundkörper besteht, der die Markierungspunkte (4) und den Lagesensor aufweist und daß an diesem Grundkörper Haltekörper aus einem flexiblen, erhärtbaren Material gebildet sind, die sich der Form von Zähnen oder dem Kiefer (1, 2) anpassen.

12. Vorrichtung zur Lagebestimmung, die für ein Verfahren nach Anspruch 9 verwendbar ist, wobei die Vorrichtung (3) in die Mundhöhle einer Person einführbar ist und so ausgebildet ist, daß sie durch Oberkiefer (2) und Unterkiefer (1) der Person in einer eindeutig reproduzierbaren Stellung gehalten werden kann, dadurch gekennzeichnet, daß die Vorrichtung (3) Markierungspunkte (4) aufweist, die sowohl mit einem bildgebenden Verfahren identifizierbar sind, als auch optisch sichtbar sind.
